(19) Europäisches Patentamt
European Patent Office
Office européen des brevets

(11) **EP 2 158 846 A2**

(12) **EUROPEAN PATENT APPLICATION**

(43) Date of publication:
**03.03.2010 Bulletin 2010/09**

(51) Int Cl.:
**A61B 8/08** (2006.01)

(21) Application number: **09011024.8**

(22) Date of filing: **27.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **29.08.2008 JP 2008222646**

(71) Applicant: **Kabushiki Kaisha Toshiba Minato-ku, Tokyo 105-8001 (JP)**

(72) Inventors:
• **Sato, Takeshi**
  **Otawara-shi**
  **Tochigi 324-8550 (JP)**
• **Mine, Yoshitaka**
  **Otawara-shi**
  **Tochigi 324-8550 (JP)**

(74) Representative: **Kramer - Barske - Schmidtchen European Patent Attorneys Landsberger Strasse 300 80687 München (DE)**

(54) **Ultrasonic diagnostic apparatus, ultrasonic image processing apparatus, and ultrasonic image processing method**

(57) When scanning a three-dimensional region while rotating a scanning plane about a predetermined axis (rotation axis), an ultrasonic diagnostic apparatus calculates a motion vector of a motion scanning plane (ultrasonic sectional layer) using ultrasonic image data in the rotation axis and corrects a positional mismatch between the scanning planes using the calculated motion vector. The motion vector is calculated and the positional mismatch between the sectional layers, using at least one frame having a cardiac time phase close to the frame of which the motion should be corrected and being spatially close to the frame, in addition to the frames at the same cardiac time phase as the frame of which the motion should be corrected.

*FIG. 3*

START

PERFORM THREE-DIMENSIONAL SCANNING OPERATION USING MTEE — S1

CREATE TWO-DIMENSIONAL IMAGE — S2

EXTRACT IMAGES AT THE SAME TIME PHASE — S3

CALCULATE MOTION VECTOR — S4

PERFORM MOTION CORRECTING PROCESS — S5

CREATE THREE-DIMENSIONAL IMAGE (THREE-DIMENSIONAL COORDINATE TRANSFORMATION/VOLUME RENDERING) — S6

DISPLAY IMAGE — S7

END

EP 2 158 846 A2

**Description**

The present invention relates to a technique of removing a noise of an image resulting from a body motion due to breathing in an ultrasonic diagnostic apparatus and the like making a four-dimensional display (real-time three-dimensional display) using a multi-plane transesophageal echocardiography (MTEE) probe, or the like.

In the ultrasonic diagnostic inspection, a heartbeat or a fetus's motion can be obtained and displayed in real time by merely touching a body surface with an ultrasonic probe, and the ultrasonic diagnostic inspection can be repeatedly carried out because of its high stability. In addition, since the system size of the ultrasonic diagnostic apparatus is smaller than those of other diagnostic apparatuses such as an X-ray diagnostic apparatus, a CT diagnostic apparatus, and an MRI diagnostic apparatus, the ultrasonic diagnostic apparatus can be easily moved to the side of a bed for inspection. For this reason, the ultrasonic diagnostic inspection can be said to be a simple diagnostic technique. The size of the ultrasonic diagnostic apparatus used in the ultrasonic diagnostic inspection varies depending on the types of functions thereof and a small-sized ultrasonic diagnostic apparatus which can be carried with one hand has been developed. Unlike the X-ray diagnostic inspection, the ultrasonic diagnostic inspection is not influenced by an exposure, and thus can be used in obstetrics and gynecology or home medical treatment and the like.

In such an ultrasonic diagnostic apparatus, a technique of scanning a three-dimensional region using an MTEE probe, creating a three-dimensional image using the acquired data, and displaying the created image in real time is known. Here, the MTEE probe is used to insert the probe into a patient's gullet to observe the heart and can also observe a section at any angle by rotating one-dimensional array vibrators. By slowly rotating the MTEE probe over 180°, the entire viewing angle of 360° can be obtained. Therefore, when a target to which the MTEE probe is applied is stationary, it is possible to construct a three-dimensional image as a still image or a real-time three-dimensional image (four-dimensional image). On the other hand, when the target, such as a heart, is moving, it is not possible to construct a correct three-dimensional image or four-dimensional image.

An example of the technique of creating and displaying a four-dimensional image of an internal organ such as a heart which is periodically moving is disclosed, for example, in US Patent No. 5,159,941. In this technique, plural frames of images at a predetermined time phase are acquired and reconstructed while rotating or moving the probe in synchronization with an ECG signal from a patient. Accordingly, it is possible to display a three-dimensional image at a predetermined time phase as if the heart is stationary.

Two-dimensional images may be continuously acquired in synchronization with the ECG signal while slowly rotating the MTEE probe at a constant velocity and a three-dimensional image may be reconstructed using the images at the same cardiac time phase out of the acquired two-dimensional images. By performing this operation on the cardiac time phases, it is possible to create a four-dimensional image representing a state where the heart is moving in one cardiac period. For example, as described in JP-A-2005-74225 and US Patent No. 6,966,878, a technique of calculating the cardiac period from an image instead of the ECG signal was suggested.

However, when the above-mentioned technique is applied to the creating and displaying of a four-dimensional image using the MTEE probe, the following problems are caused.

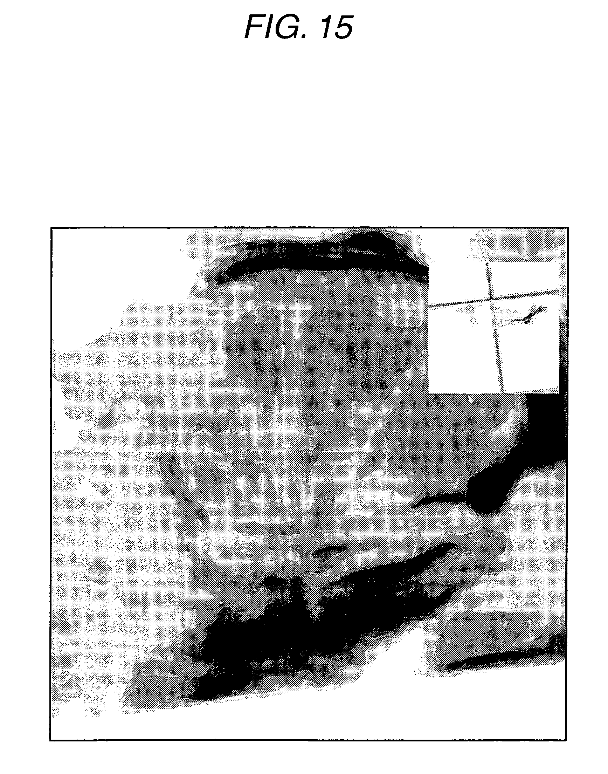

That is, in creating and displaying a four-dimensional image using the MTEE probe, 1080 pieces of two-dimensional images are actually required to obtain a clear four-dimensional image. Accordingly, for example, a scanning time of 36 seconds is required, when the number of heartbeats is 60 beats/min, the number of frames of sectional layer images to be scanned is 30 frames/sec, and the rotation angle of the probe for each sectional layer image is 0.167°. When the heart moves due to a patient's breathing or the like in the meantime, the strain of a heart shape, shown in FIG. 15, is caused in the four-dimensional image.

This problem is caused by the assumption that a cardiac wall or a valve has the same three-dimensional position and shape at the same cardiac time phase. This assumption is correct to a certain extent, when no arrhythmia occurs and when the entire heart does not move due to breathing. However, when no arrhythmia occurs but a patient breathes, the entire heart moves and thus the above-mentioned assumption is not established. Since a patient cannot stop breathing for 36 seconds in a state where the patient swallows an endoscope, this technique has a great problem.

The invention is contrived in view of the above-mentioned problem. An object of the invention is to provide an ultrasonic diagnostic apparatus, an ultrasonic image processing apparatus, and an ultrasonic image processing method capable of removing a noise resulting from a body motion due to breathing in acquiring plural two-dimensional ultrasonic images while rotating an ultrasonic scanning plane about an axis and reconstructing a three-dimensional image using the acquired two-dimensional ultrasonic images.

According to another aspect of the present invention, there is provided that an ultrasonic diagnostic apparatus which comprises: a multi-plane transesophageal echocardiography probe; an data acquisition unit which acquires ultrasonic image data of multi-plane corresponding to a plurality of scan planes by using the multi-plane transesophageal echocardiography probe; and a correction unit configured to correct a positional mismatch among the multi-plane on the basis of motion vectors of scanning planes scanned by the multi-plane transesophageal echocardiography probe.

According to yet another aspect of the present invention, there is provided that an ultrasonic image processing apparatus which comprises: a memory unit configured to store data of a plurality of frames of ultrasonic images, which are acquired by three-dimensionally scanning a diagnosis target with ultrasonic waves while rotating a scanning plane about a rotation axis, corresponding to a plurality of scanning planes; and a correction unit configured to correct a positional mismatch between the frames on the basis of data, corresponding to the rotation axis, of the data of the plurality of frames of ultrasonic images.

According to yet another aspect of the present invention, there is provided that an ultrasonic image processing apparatus which comprises: a store unit which stores ultrasonic image data of multi-plane which is acquired by using a multi-plane transesophageal echocardiography probe and corresponds to a plurality of scan planes; and a correction unit configured to correct a positional mismatch among the multi-plane on the basis of motion vectors of scanning planes scanned by the multi-plane transesophageal echocardiography probe.

According to yet another aspect of the present invention, there is provided that an ultrasonic image processing method which comprises: acquiring data of a plurality of frames of ultrasonic images corresponding to a plurality of scanning planes by three-dimensionally scanning a diagnosis target with ultrasonic waves while rotating the scanning planes about a rotation axis; and correcting a positional mismatch between the frames on the basis of data, corresponding to the rotation axis, of the data of the plurality of frames of ultrasonic images.

According to another aspect of the present invention, there is provided that an ultrasonic image processing method which comprises: acquiring ultrasonic image data of multi-plane corresponding to a plurality of scan planes by using a multi-plane transesophageal echocardiography probe; and correcting a positional mismatch among the multi-plane on the basis of motion vectors of scanning planes scanned by the multi-plane transesophageal echocardiography probe.

The invention can be more fully understood from the following detailed description when taken in conjunction with the accompanying drawings, in which:

FIG. 1 is a block diagram illustrating the configuration of an ultrasonic diagnostic apparatus according to an embodiment of the invention.

FIGS. 2A and 2B are diagrams illustrating the configuration of an ultrasonic probe 12.

FIG. 3 is a flowchart illustrating a flow of processes in removing a noise resulting from a body motion.

FIG. 4 is a diagram illustrating details of three-dimensional scanning using an MTEE.

FIGS. 5A, 5B, 5C, and 5D are diagrams illustrating a process of extracting two-dimensional images at the same cardiac time phase.

FIGS. 6A and 6B are diagrams illustrating the concept of a motion vector calculating method.

FIG. 7 is a diagram illustrating the concept of the motion vector calculating method.

FIG. 8 is a diagram illustrating the concept of the motion vector calculating method.

FIG. 9 is a diagram illustrating a process of correcting a motion of a scanning plane due to a body motion.

FIGS. 10A and 10B are diagrams illustrating the process of correcting a motion of a scanning plane due to a body motion.

FIGS. 11A and 11B are diagrams illustrating a process of creating a three-dimensional image.

FIG. 12 is a flowchart illustrating a flow of processes in removing a noise resulting from a body motion according to a second embodiment of the invention.

FIG. 13 is a diagram illustrating a motion vector calculating process according to the second embodiment.

FIG. 14 is a flowchart illustrating a flow of processes in removing a noise resulting from a body motion according to a third embodiment of the invention.

FIG. 15 is a diagram illustrating a prior art.

Hereinafter, first to third embodiments of the invention will be described with reference to the accompanying drawings. In the following description, elements having substantially like functions and configurations are referenced by like reference numerals and signs and their descriptions are omitted other than needed.

First Embodiment

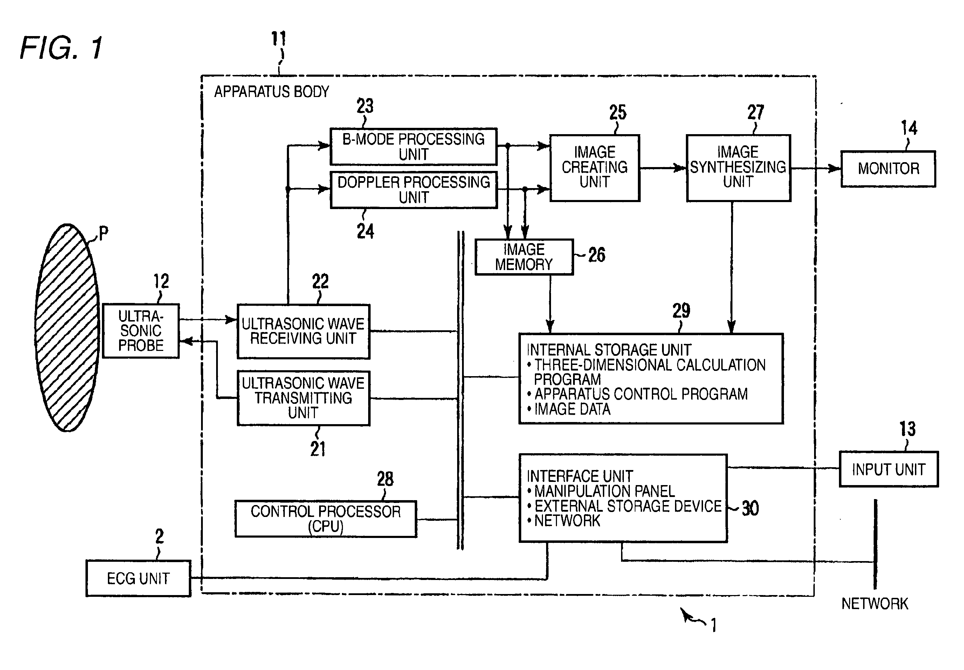

FIG. 1 is a block diagram illustrating the configuration of an ultrasonic diagnostic apparatus according to a first embodiment of the invention. As shown in the drawing, an ultrasonic diagnostic apparatus 1 includes: an ultrasonic probe 12; an input unit 13; a monitor 14; an ultrasonic wave transmitting unit 21; an ultrasonic wave receiving unit 22; a B-mode processing unit 23; a Doppler processing unit 24; an image creating unit 25; an image memory 26; an image synthesizing unit 27; a control processor (CPU) 28; an internal storage unit 29; and an interface unit 30. Hereinafter, the function of each element will be described. The ultrasonic diagnostic apparatus 1 is connected to an ECG unit for measuring an ECG signal (electrocardiographic signal) of a sample.

The ultrasonic probe 12 generates ultrasonic waves on the basis of a driving signal from the ultrasonic wave transmitting unit 21, and includes plural piezoelectric vibrators converting ultrasonic waves reflected from a sample into electric signals, a matching layer disposed in the piezoelectric vibrators, and a backing member and the like for preventing the ultrasonic waves from being transmitted backwards from the piezoelectric vibrators. When the ultrasonic waves are transmitted from the ultrasonic probe 12 to a sample P, the transmitted ultrasonic waves are sequentially reflected by surfaces of a body tissue with discontinuous acoustic impedance and are received by the ultrasonic probe 12 in the form of echo signals. The amplitudes of the echo signals are dependent on the difference in acoustic impedance between the discontinuous surfaces. In addition, when the transmitted ultrasonic pulses are reflected by a moving blood stream, the surface of a cardiac wall, or the like, a frequency shift of the echo signal occurs depending on velocity components of a moving object in an ultrasonic wave transmitting direction by the Doppler effect.

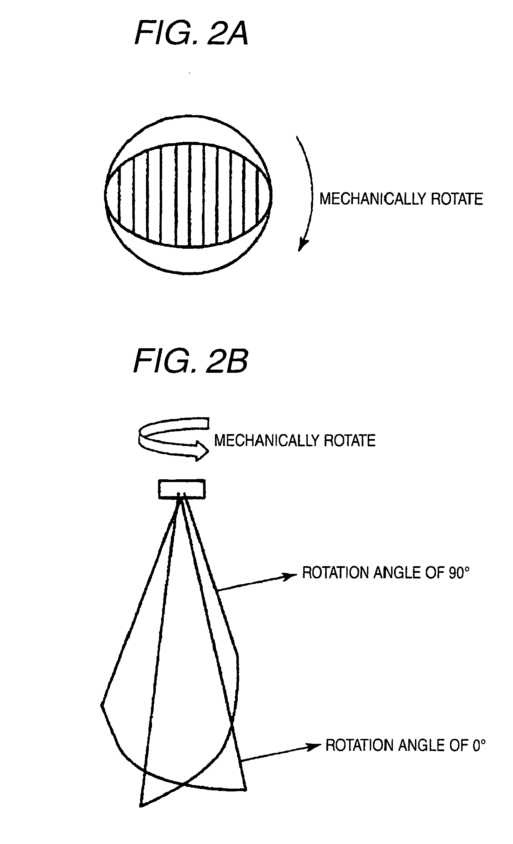

The ultrasonic probe 12 of the ultrasonic diagnostic apparatus can rotate a scanning plane about a predetermined axis. Typically, a two-dimensional array probe capable of scanning a three-dimensional region with ultrasonic waves under the electrical control using two-dimensional vibrators two-dimensionally arranged or an MTEE probe can be employed as the ultrasonic probe. Here, the MTEE probe (multi-plane transesophageal echocardiography probe) is used to observe a section at any angle by rotating one-dimensional array vibrators and to observe the heart by inserting the probe into a patient's gullet, as shown in FIGS. 2A and 2B.

The input unit 13 is connected to the apparatus body 11 and includes various switches and buttons, a track ball, a mouse, a keyboard, and the like, which are used to allow an operator to input various instructions, conditions, ROI (region of interest) setting instructions, image quality condition setting instructions, and the like to the apparatus body 11. For example, when the operator manipulates an end button or a freeze button of the input unit 13, the ultrasonic wave transmitting and receiving operations end and the ultrasonic diagnostic apparatus is changed to a pause state.

The monitor 14 displays morphological information (general B-mode image) inside a biological body, blood stream information (an average-velocity image, a distribution image, a power image, and the like), and combinations thereof as an image on the basis of a video signal from the image creating unit 25.

The ultrasonic wave transmitting unit 21 includes a trigger generating circuit, a delay circuit, and a pulser circuit and the like, which are not shown in the drawings. The pulser circuit repeatedly generates rated pulses at a predetermined rated frequency fr Hz (period; 1/fr second) so as to form the transmitted ultrasonic waves. The delay circuit converges the ultrasonic waves into a beam every channel and gives a delay time required for determining the transmitting directivity to the rated pulses. The trigger generating circuit applies a driving pulse to the probe 12 at a timing based on the rated pulse.

The ultrasonic wave transmitting unit 21 has a function of instantaneously changing a transmission frequency and a transmission driving voltage so as to carry out a predetermined scanning sequence in accordance with an instruction of the control processor 28. Particularly, the changing of the transmission driving voltage is carried out by a linear amplifier type transmitting circuit capable of instantaneously switching the value or a mechanism electrically switching plural power supply units.

The ultrasonic wave receiving unit 22 includes an amplifier circuit, an A/D converter, an adder and the like, which are not shown in the drawings. The amplifier circuit amplifies the echo signal input from the probe 12 for each channel. The A/D converter gives a time required for determining the receiving directivity to the amplified echo signal, and the amplified echo signal is subjected to an adding process by the adder. By means of the adding process, a reflected component in the direction corresponding to the receiving directivity of the echo signal is emphasized, and a synthetic beam for transmitting and receiving ultrasonic waves is formed on the basis of the receiving directivity and the transmitting directivity.

The B-mode processing unit 23 receives the echo signal from the ultrasonic wave transmitting unit 21 and performs a log amplifying process, an envelope detecting process, and the like so as to create data in which the signal strength is expressed by luminance. The data is transmitted to the image creating unit 25 and is displayed as a B-mode image, in which the strength of the reflected wave is expressed by luminance, on the monitor 14.

The Doppler processing unit 24 frequency-analyzes the velocity information on the basis of the echo signal transmitted from the ultrasonic wave transmitting unit 21 and extracts an echo component of a blood stream, a tissue, or a contrast agent using the Doppler effect so as to obtain the blood stream information such as an average velocity, a distribution, and a power at plural points.

In general, the image creating unit 25 converts (scan-converts) a scanning-line signal stream scanned with ultrasonic waves into a scanning-line signal stream in the general video format represented by television and creates an ultrasonic diagnostic image as a display image. The image creating unit 25 performs a process (process of removing a noise resulting from a body motion) of a function of removing a noise resulting from a body motion to be described later. Data not inputted yet to the image creating unit 25 can be referred to as "raw data". A volume rendering processor may have any configuration of CPU, GPU, DSP, ASIC, and the like.

The image memory (cine memory) 26 is a memory configured to store ultrasonic images corresponding to plural frames, for example, just before being frozen. It is also possible to display an ultrasonic video image by continuously displaying (cine-displaying) the images stored in the image memory 26.

The image synthesizing unit 27 synthesizes the image received from the image creating unit 25 with texts or scales of various parameters and outputs the resultant image as a video signal to the monitor 14.

The control processor 28 has a function of an information processing device (computer) and controls the operations of the ultrasonic diagnostic apparatus body. The control processor 28 reads out an exclusive program for realizing the function of removing a noise resulting from a body motion and a control program for performing the creation and display of a predetermined image from the internal storage unit 29, and loads the programs in its memory so as to make calculation control and the like of various processes. The ECG signal from the ECG unit 2 is input to the control processor 28 via the interface unit 30. The control processor 28 determines to what cardiac time phase each frame obtained by the ultrasonic scanning corresponds on the basis of the input ECG signal.

The internal storage unit 29 stores a predetermined scanning sequence, an exclusive program for performing the function of removing a noise resulting from a body motion according to the embodiments, a control program for performing the creating and displaying of an image, diagnostic information (patient ID, doctor's opinion, and the like), a diagnostic protocol, transmitting and receiving conditions, and a CFAR processing and controlling program, a body mark creating program, and other data groups. If necessary, the internal storage unit 29 is also used to store images in the image memory 26. The data in the internal storage unit 29 can be transmitted to an external peripheral device via the interface unit 30.

The interface unit 30 is an interface with the input unit 13, a network, and a new external storage device (not shown). The data such as the ultrasonic image, the analysis result, or the like, obtained by the apparatus can be transmitted to other devices via the network by the interface unit 30. Function of Removing Noise Resulting from Body Motion

The function of removing a noise resulting from a body motion in the ultrasonic diagnostic apparatus 1 will be described now. This function serves to provide an ultrasonic image with high image quality by removing a noise or strain resulting from a body motion due to breathing, or the like, when scanning a three-dimensional region while rotating an ultrasonic scanning plane about a predetermined axis. This function may be applied to an ultrasonic image processing apparatus which is embodied by installing an exclusive program, for example, in a work station or a personal computer.

In this embodiment, for the purpose of specific explanation, it is assumed that the diagnosis target is a heart and the three-dimensional region thereof is canned with the MTEE probe while rotating the ultrasonic scanning plane. However, the technical spirit of the invention is not limited to the heart, but may be applied to a case, for example, where the diagnosis target is an abdominal region such as the liver. In this embodiment, a slight variation in the heartbeat period is permitted but a great variation in the heartbeat period like arrhythmia is excluded.

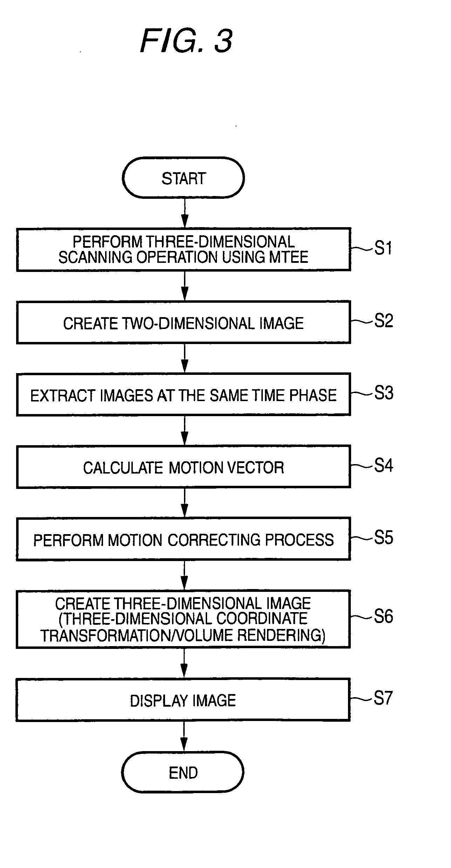

FIG. 3 is a flowchart illustrating a flow of processes corresponding to the function of removing a noise resulting from the body motion (the process of removing a noise resulting from the body motion). The processing details of the steps will be described now. Three-dimensional Scanning using MTEE and Creation of Two-dimensional Image: Steps S1 and S2

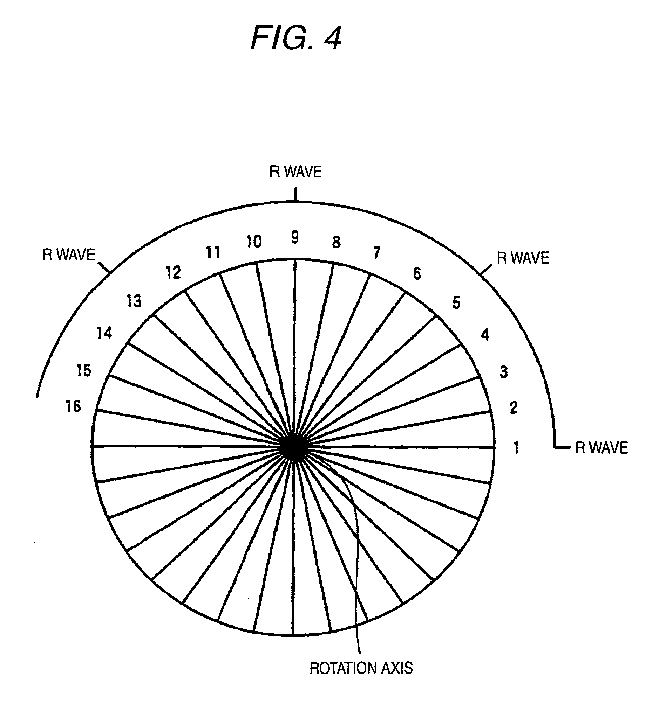

First, a three-dimensional scanning operation is carried out in the range of 180° under the control of the control processor 28 using an ECG signal as a trigger while rotating plural ultrasonic vibrators of the ultrasonic probe 12 (MTEE probe) about the rotation axis for each scanning (while changing the rotation angle), whereby echo signals (here, the echo signals corresponding to 16 frames, for example, as shown in FIG. 4) corresponding to plural frames over plural heartbeats are acquired (step S1). In this scanning, the signal temporally and spatially varies every frame. Information (cardiac time phase information) representing the cardiac time phases corresponding to the frames is acquired on the basis of the ECG signal by the control processor 28 and is automatically stored, for example, in the internal storage unit 29.

Plural frames of echo signals acquired by the scanning are sent to the B-mode processing unit 23 via the ultrasonic wave receiving unit 22 for each frame. The B-mode processing unit 23 creates B-mode image data of each frame.

The image creating unit 25 creates a two-dimensional image of each frame on the basis of the B-mode image data of each frame (step S2). Extraction of Two-dimensional Image at the Same Cardiac Time Phase: Step S3

The image creating unit 25 extracts the two-dimensional images at the same cardiac time phase (with the same cardiac period) using the cardiac time phase information and acquires plural two-dimensional images with three-dimensionally different positions (spatially different positions) at the time phases (step S3).

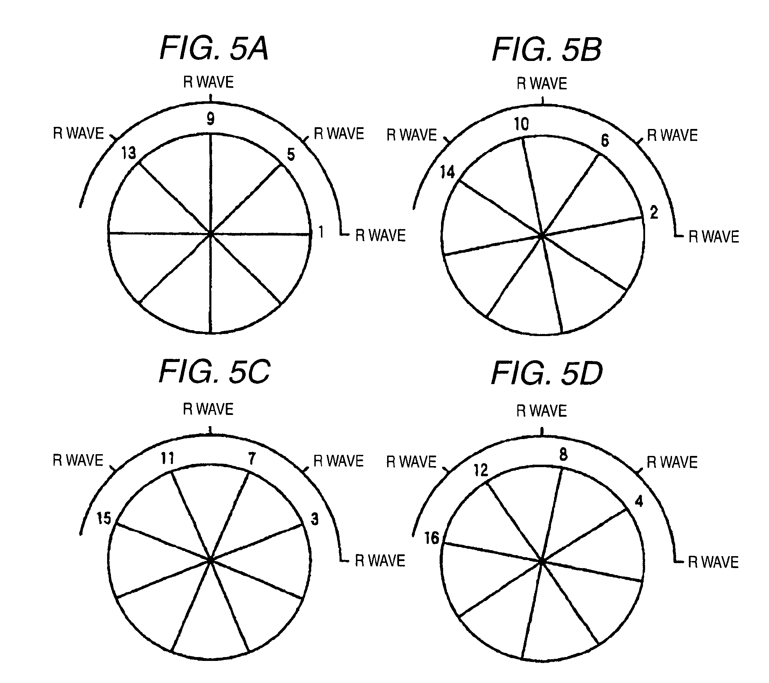

FIGS. 5A, 5B, 5C, and 5D are diagrams illustrating the process of step S3. That is, out of 16 acquired two-dimensional images shown in FIG. 4, FIG. 5A shows that two-dimensional images 1, 5, 9, and 13 at the first cardiac time phase corresponding to the detection of R wave are extracted, FIG. 5B shows that two-dimensional images 2, 6, 10, and 14 at the second cardiac time phase corresponding to the detection of R wave are extracted, FIG. 5C shows that two-dimensional images 3, 7, 11, and 15 at the third cardiac time phase corresponding to the detection of R wave are extracted, and FIG. 5D shows that two-dimensional images 4, 8, 12, and 16 at the four cardiac time phase corresponding to the detection of R wave are extracted.

Calculation of Motion Vector: Step S4

The image creating unit 25 calculates a motion vector of each frame (each scanning plane) (step S4). The method of calculating a motion vector is as follows.

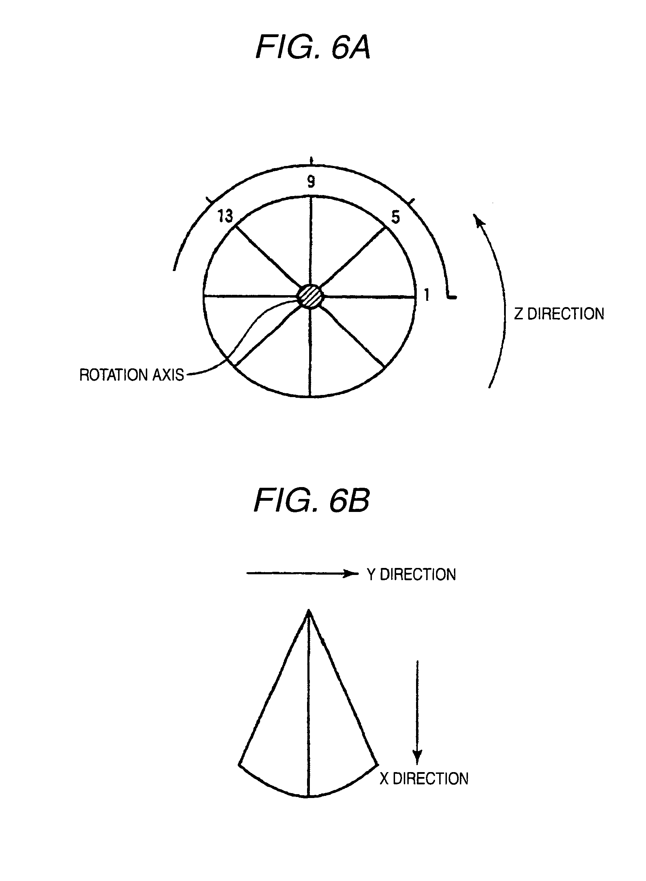

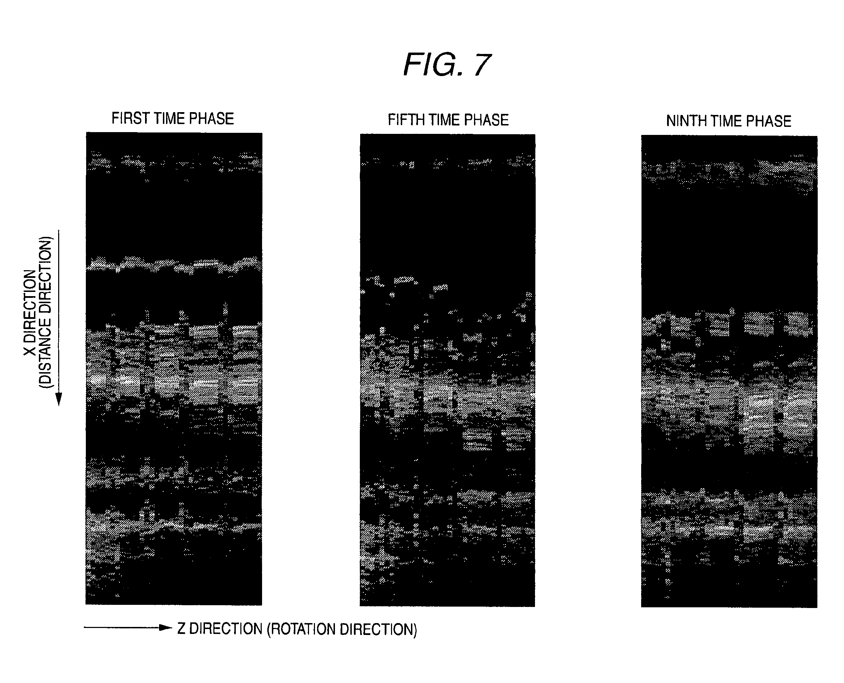

FIGS. 6A and 6B and FIG. 7 are diagrams illustrating the concept of the method of calculating a motion vector. As shown in FIGS. 6A and 6B, the center axes of the data of the frames in volumes at the same cardiac time phase pass through the same lines. Therefore, the data in the center axes of the frames will have the same value when there is no body motion due to breathing, or the like. As shown in FIG. 7, when actual images in the center axes of the frames are arranged (where the horizontal axis represents the frame number (rotation direction=Z direction) and the vertical axis represents the distance direction (X direction)), it can be seen that the images are changed due to breathing about every four seconds.

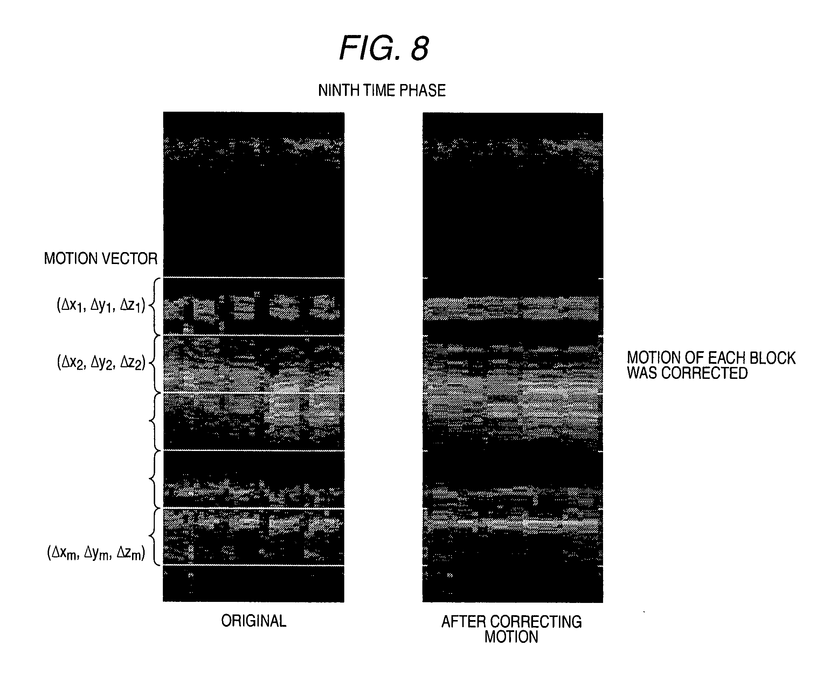

In order to correct this motion, the image creating unit 25 determines a reference frame k (for example, #1 frame), calculates correlation coefficients by shifting the reference frame in the X, Y, and Z directions from the center axis of the k-th frame and the center axis of the n-th frame (for example, frames of #5, #9, and #13), and sets the position ($\Delta$x, $\Delta$y, $\Delta$z) having the maximum as a motion vector. At this time, as shown in FIG. 8, it is preferable that the line is divided in the X direction into blocks and the process is performed on each block (where the ninth time phase is exemplified in FIG. 8). It is preferable that a frame having no variation in motion is selected as the reference frame k. The position having the minimum sum of absolute differences or the minimum sum of squared differences may be used instead of the correlation coefficient.

Motion Correcting Process: Step S5

The image creating unit 25 calculates the motion vector of the entire frame from the motion vectors of the blocks of every frame and corrects the motions of each frame using the motion vector (step S5).

It can be seen from the motion vectors of the blocks where the center positions of the blocks are displaced. Accordingly, the image creating unit 25 calculates the positions of the observed sectional surface when there is no motion (accurately, in the state of the reference frame) on the basis of the plural motion vectors corresponding to the blocks of each frame. That is, the image creating unit 25 calculates (6 in total) parameters of parallel shift and rotation angle about x, y, and z axes, which are expressed by Expression 1, using the least square method on the basis of the plural motion vectors corresponding to the blocks of each frame.

Expression 1

$$\begin{pmatrix} x_1' \\ y_1' \\ z_1' \end{pmatrix} = \begin{pmatrix} \cos\theta_z & -\sin\theta_z & 0 \\ \sin\theta_z & \cos\theta_z & 0 \\ 0 & 0 & 1 \end{pmatrix} \begin{pmatrix} \cos\theta_y & 0 & \sin\theta_y \\ 0 & 1 & 0 \\ -\sin\theta_y & 0 & \cos\theta_y \end{pmatrix} \begin{pmatrix} 1 & 0 & 0 \\ 0 & \cos\theta_x & -\sin\theta_x \\ 0 & \sin\theta_x & \cos\theta_x \end{pmatrix} \begin{pmatrix} x_1 \\ y_1 \\ z_1 \end{pmatrix} + \begin{pmatrix} x_0 \\ y_0 \\ z_0 \end{pmatrix}$$

Here,

$$\begin{pmatrix} x_1 \\ y_1 \\ z_1 \end{pmatrix}$$

represents the position of the reference frame,

$$\begin{pmatrix} x_1' \\ y_1' \\ z_1' \end{pmatrix}$$

represents the position having the maximum correlation coefficient, $\theta_X$, $\theta_y$, and $\theta_z$ represent the rotation angles, and $x_0$, $y_0$, and $z_0$ represent the distances of the parallel shift.

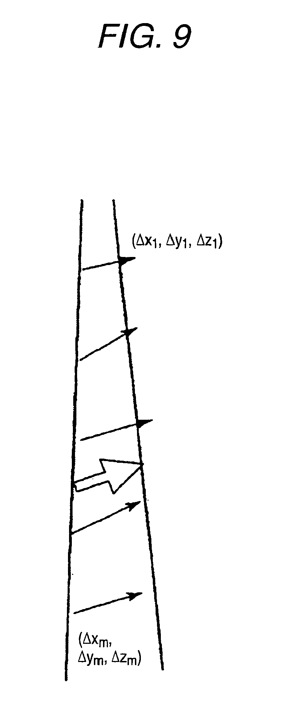

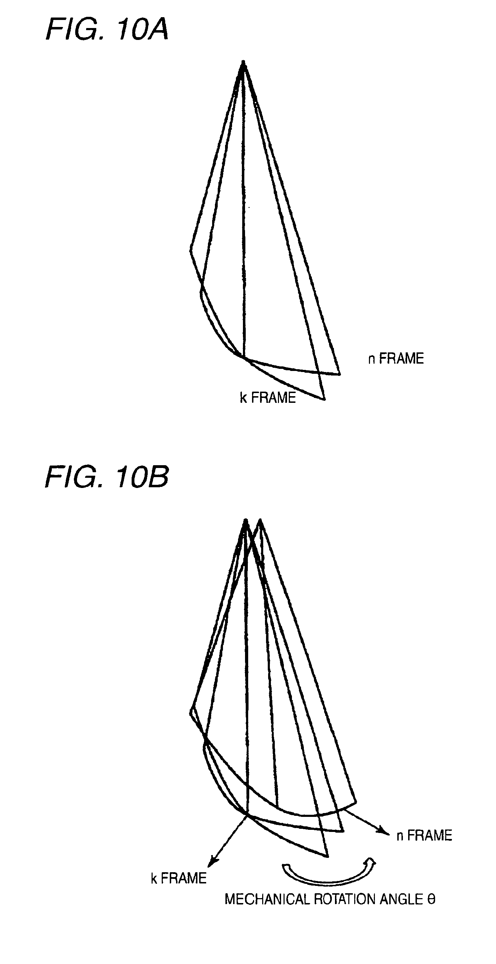

Then, the image creating unit 25 performs the motion correcting process by shifting the positions of the points in the observed sectional surface, as shown in FIG. 9, by the use of Expression 1 where the parameters are determined for each frame, so that the observed sectional surface shown in FIG. 10A is located at the position (corresponding to the reference frame) shown in FIG. 10B when it is assumed that there is no body motion.

In this embodiment, it is assumed that no strain exists and that the motion is limited to the parallel shift and the rotation. At least two motion vectors are required to calculate the parameters of Expression 1. However, in this embodiment, it is assumed that the parameters are estimated from the plural motion vectors by the use of the least square method in order to enhance the precision.

Creation of Three-dimensional Image: Step S6

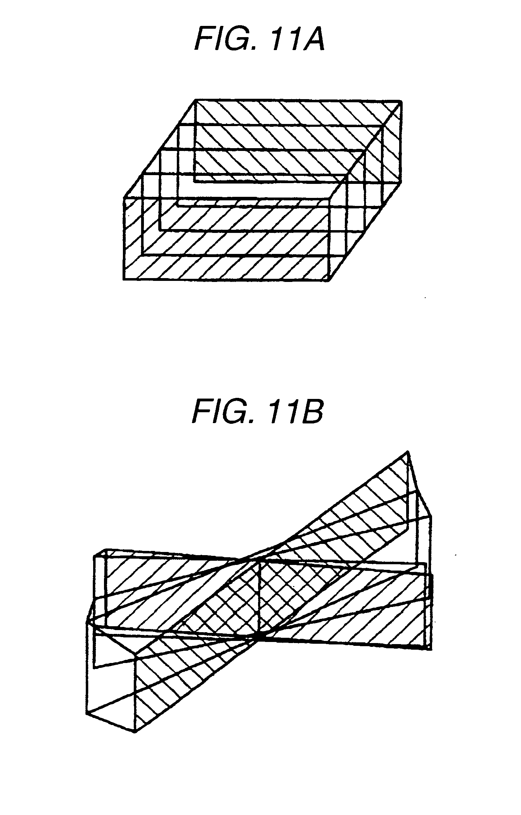

The image creating unit 25 performs the volume rendering on the basis of the new sectional position acquired in the motion correcting process of step S5 to create a three-dimensional image (step S6). At this time, the three-dimensional coordinate transformation is complex, because the sectional planes are not regularly arranged. However, the three-dimensional coordinate transformation is possible by dividing the spatial positions before the coordinate transformation formed by two adjacent frames, as shown in FIG. 11A, and the positions in the three-dimensional space as shown in FIG. 11B into plural polygons.

Image Display: Step S7

The image synthesizing unit 27 synthesizes the images received from the image creating unit 25 along with the character or scale information of various parameters. Then, the control processor 28 controls the display unit 14 to display the three-dimensional image synthesized with the character information, and the like, in a predetermined form.

Advantage

According to the above-mentioned configuration, the following advantages can be obtained.

In the ultrasonic diagnostic apparatus according to this embodiment, when a three-dimensional region is canned while rotating the scanning plane about a predetermined axis (rotation axis), the motion vector of the motion scanning plane (ultrasonic sectional layer) is calculated using the ultrasonic image data in the rotation axis and the positional mismatch between the scanning planes is corrected using the motion vector. Therefore, even when a body motion due to breathing, or the like, occurs in the course of the ultrasonic scanning, it is possible to remove the influence of the body motion on the image and thus to provide a high-quality image without any strain. As a result, when an image of a heart, or the like, is four-dimensionally displayed, it is possible to provide a high-reliability image at relatively low cost without imposing a burden on a patient or an operator.

Second Embodiment

A second embodiment of the invention will be described now. In order to enhance the resolution in the rotation direction (Z direction), an ultrasonic diagnostic apparatus 1 according to this embodiment also uses the frames corresponding to sectional surfaces, which are temporally close (that is, of which the cardiac time phase is close) and spatially close, to correct the motion in addition to the frames corresponding to the sectional surfaces having the same cardiac time phase.

A process of removing noises corresponding to the sectional surfaces according to this embodiment will be described now.

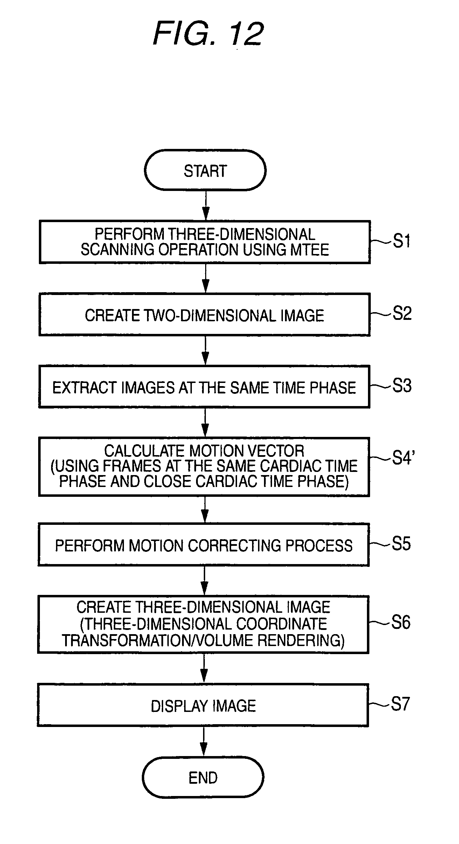

FIG. 12 is a flowchart illustrating a flow of the process of removing a noise resulting from a body motion according to this embodiment. The drawing is different from FIG. 3 only in step S4' and is substantially equal in the details of the other steps. Only the different details will be described. Calculation of Motion Vector: step S4'

The image creating unit 25 calculates the motion vector of each frame using the frames having the same cardiac time phase and the frames having a close cardiac time phase (step S4').

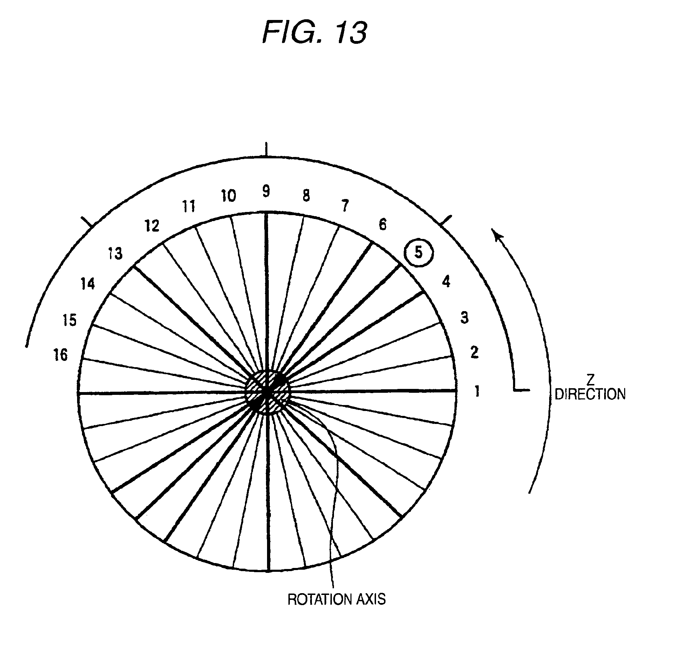

That is, in FIG. 13, for example, when the motion of sectional surface 5 having the first cardiac time phase (including sectional surfaces of #1, #5, #9, and #13 (see FIG. 5)) is corrected, at least one sectional surface (for example, #4, #6, and the like) having a cardiac time phase different but close is also used to calculate the motion vector of sectional surface 5, in addition to the frames of #1, #5, #9, and #13 having the same cardiac time phase. The specific calculation method is the same as the first embodiment.

According to the above-mentioned configuration, at the time of calculating a motion vector, at least one frame having a cardiac time phase close to the frame of which the motion should be corrected and being spatially close thereto in addition to the frames having the same cardiac time phase as the frame of which the motion should be corrected is used to calculate the motion vector, whereby the positional mismatch between the sectional surfaces is corrected. Therefore, it is possible to improve the resolution in the rotation direction, thereby more effectively correcting the motion.

Third Embodiment

A third embodiment of the invention will be described now. In an ultrasonic diagnostic apparatus 1 according to this embodiment, for example, a diagnosis target is a site such as the liver not having a periodic motion and thus the ECG signal is not required.

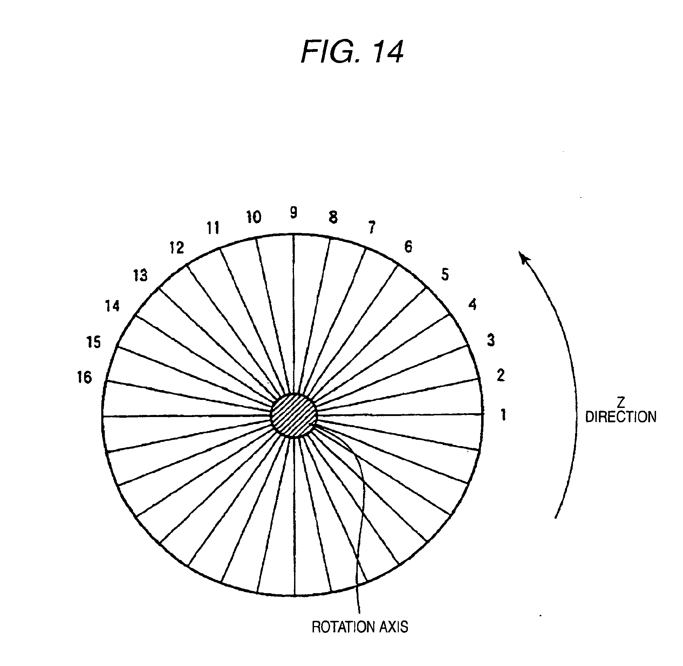

FIG. 14 is a diagram illustrating the process of removing a noise resulting from a body motion according to this embodiment. In this embodiment, the diagnosis target does not voluntarily move. Therefore, as shown in FIG. 14, the data in the rotation axes of all the sectional surfaces (that is, the sectional surfaces of #1 to #16) will be equal to each other when there is no body motion due to breathing, or the like. Accordingly, the ultrasonic diagnostic apparatus 1 according to this embodiment uses the data in the rotation axes of all the sectional surfaces when the motion vectors of the frames are calculated.

According to this configuration, it is possible to further improve the resolution in the rotation direction, thereby more effectively correcting the motion.

The invention is not limited to the above-mentioned embodiments, but the elements may be modified and embodied without departing from the spirit and scope of the invention at the time of putting the invention into practice. Specific modified examples are as follows.

(1) The functions according to the embodiments can be embodied by installing programs for carrying out the functions in a computer such as a work station and loading the programs into the memory. At this time, the programs allowing the computer to perform the methods may be recorded in a recording medium such as a magnetic disk (such as a floppy (registered trademark) disk and a hard disk), an optical disk (such as a CD-ROM and a DVD), and a semiconductor memory and may be then distributed.

(2) In the above-mentioned embodiments, the motion is defined by the constant parallel shift and rotation angle of a sectional surface, but the sectional surface may be divided into plural blocks and the parallel shift and the rotation may be made every block. It is possible to consider the strain.

(3) In the above-mentioned embodiments, the B-mode image data as raw data are transformed into two-dimensional coordinates, are subjected to the motion vector calculating process and the motion correcting process, and are then subjected to the three-dimensional coordinate transformation (creation of a three-dimensional image). On the contrary, without performing the two-dimensional coordinate transformation, the B-mode image data as the raw data are subjected to the motion vector calculating process and the motion correcting process and are then subjected to the three-dimensional coordinate transformation.

The invention may be modified in various forms by properly combining plural elements provided in the above-mentioned embodiments. For example, several elements may be deleted from all the elements of one embodiment. The elements of different embodiments may be properly combined.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

**Claims**

1. An ultrasonic diagnostic apparatus (1) **characterized by** comprising:

an ultrasonic image data acquiring unit (12, 21, 22, 23, 24) adapted to three-dimensionally scan a diagnosis target with ultrasonic waves while rotating a scanning plane about a rotation axis and to acquire data of a plurality of frames of ultrasonic images corresponding to a plurality of scanning planes;
a correction unit (25, 28) adapted to correct a positional mismatch between the frames on the basis of data, corresponding to the rotation axis, of the data of the plurality of frames of ultrasonic images; and
an image creating unit (25) configured to create a three-dimensional image using the data of the plurality of frames of ultrasonic images of which the positional mismatch is corrected.

2.  An ultrasonic diagnostic apparatus (1) **characterized by** comprising:

a multi-plane transesophageal echocardiography probe (12);
a data acquisition unit adapted to acquire ultrasonic image data of multi-plane corresponding to a plurality of scan planes by using the multi-plane transesophageal echocardiography probe (12); and
a correction unit (25, 28) adapted to correct a positional mismatch among the multi-plane on the basis of motion vectors of scanning planes scanned by the multi-plane transesophageal echocardiography probe (12).

3.  An ultrasonic image processing apparatus **characterized by** comprising:

a memory unit (29) adapted to store data of a plurality of frames of ultrasonic images, which are acquired by three-dimensionally scanning a diagnosis target with ultrasonic waves while rotating a scanning plane about a rotation axis, corresponding to a plurality of scanning planes; and
a correction unit (25, 28) adapted to correct a positional mismatch between the frames on the basis of data, corresponding to the rotation axis, of the data of the plurality of frames of ultrasonic images.

4.  The ultrasonic image processing apparatus according to claim 1 or 3, **characterized in that** the diagnosis target is a heart, and
wherein the correction unit (25, 28) is adapted to calculate a motion vector of each frame on the basis of the data, corresponding to the rotation axis, of the ultrasonic image data at the same cardiac time phase as the frame to be corrected out of the data of the plurality of ultrasonic images and to correct the positional mismatch between the frames using the motion vectors.

5.  The ultrasonic image processing apparatus according to claim 1 or 3, **characterized in that** the diagnosis target is a heart, and
wherein the correction unit (25, 28) is adapted to calculate a motion vector of each frame on the basis of the data, corresponding to the rotation axis, of the ultrasonic image data at the same cardiac time phase as the frame to be corrected and the data, corresponding to the rotation axis, of the ultrasonic image data corresponding to at least one frame temporally and spatially close to the frame to be corrected out of the data of the plurality of ultrasonic images and to correct the positional mismatch between the frames using the motion vectors.

6.  The ultrasonic image processing apparatus according to claim 1 or 3, **characterized in that** the diagnosis target is an internal organ other than a heart, and
wherein the correction unit (25, 28) is adapted to calculate motion vectors using the data, corresponding to the rotation axis, of the frame to be corrected and the data, corresponding to the rotation axis, of the frames other than the frame to be corrected and to correct the positional mismatch between the frames using the motion vectors.

7.  The ultrasonic image processing apparatus according to any of claims 4 to 6, **characterized in that** the correction unit (25, 28) is adapted to divide each ultrasonic image data into a plurality of blocks, calculate a motion vector of each block of each frame, calculate a motion vector of each frame using the motion vectors of the blocks, and correct the positional mismatch between the frames using the calculated motion vector.

8.  The ultrasonic image processing apparatus according to any of claims 4 to 6, **characterized in that** the correction unit (25, 28) is adapted to divide each ultrasonic image data into a plurality of blocks, calculate a motion vector of each block of each frame, and correct the positional mismatch between the frames by correcting the positional mismatch between the blocks of the frames using the motion vectors of the blocks.

9.  The ultrasonic image processing apparatus according to claim 1 or any of claims 3 to 8, **characterized in that** the correction unit (25, 28) is adapted to calculate motion vectors after converting the data of the plurality of ultrasonic images into two-dimensional coordinates.

10. An ultrasonic image processing apparatus (1) **characterized by** comprising:

a store unit (29) adapted to store ultrasonic image data of multi-plane which is acquired by using a multi-plane transesophageal echocardiography probe (12) and corresponds to a plurality of scan planes; and
a correction unit (25, 28) adapted to correct a positional mismatch among the multi-plane on the basis of motion vectors of scanning planes scanned by the multi-plane transesophageal echocardiography probe (12).

**11.** An ultrasonic image processing method **characterized by** comprising:

acquiring data of a plurality of frames of ultrasonic images corresponding to a plurality of scanning planes by three-dimensionally scanning a diagnosis target with ultrasonic waves while rotating the scanning planes about a rotation axis; and

correcting a positional mismatch between the frames on the basis of data, corresponding to the rotation axis, of the data of the plurality of frames of ultrasonic images.

**12.** An ultrasonic image processing method **characterized by** comprising:

acquiring ultrasonic image data of multi-plane corresponding to a plurality of scan planes by using a multi-plane transesophageal echocardiography probe (12); and

correcting a positional mismatch among the multi-plane on the basis of motion vectors of scanning planes scanned by the multi-plane transesophageal echocardiography probe (12).

# FIG. 1

APPARATUS BODY (11)

B-MODE PROCESSING UNIT (23) → IMAGE CREATING UNIT (25) → IMAGE SYNTHESIZING UNIT (27) → MONITOR (14)

DOPPLER PROCESSING UNIT (24)

IMAGE MEMORY (26)

P

ULTRA-SONIC PROBE (12)

ULTRASONIC WAVE RECEIVING UNIT (22)

ULTRASONIC WAVE TRANSMITTING UNIT (21)

INTERNAL STORAGE UNIT (29)
• THREE-DIMENSIONAL CALCULATION PROGRAM
• APPARATUS CONTROL PROGRAM
• IMAGE DATA

CONTROL PROCESSOR (CPU) (28)

INTERFACE UNIT (30)
• MANIPULATION PANEL
• EXTERNAL STORAGE DEVICE
• NETWORK

INPUT UNIT (13)

ECG UNIT (2)

NETWORK

1

EP 2 158 846 A2

## FIG. 2A

MECHANICALLY ROTATE

## FIG. 2B

MECHANICALLY ROTATE

ROTATION ANGLE OF 90°

ROTATION ANGLE OF 0°

# FIG. 3

START

PERFORM THREE-DIMENSIONAL SCANNING OPERATION USING MTEE ～S1

CREATE TWO-DIMENSIONAL IMAGE ～S2

EXTRACT IMAGES AT THE SAME TIME PHASE ～S3

CALCULATE MOTION VECTOR ～S4

PERFORM MOTION CORRECTING PROCESS ～S5

CREATE THREE-DIMENSIONAL IMAGE (THREE-DIMENSIONAL COORDINATE TRANSFORMATION/VOLUME RENDERING) ～S6

DISPLAY IMAGE ～S7

END

## FIG. 4

FIG. 5B

FIG. 5A

FIG. 5D

FIG. 5C

## FIG. 6A

## FIG. 6B

*FIG. 7*

NINTH TIME PHASE

FIFTH TIME PHASE

FIRST TIME PHASE

X DIRECTION (DISTANCE DIRECTION)

Z DIRECTION (ROTATION DIRECTION)

## FIG. 8

NINTH TIME PHASE

MOTION VECTOR

$(\Delta x_1, \Delta y_1, \Delta z_1)$

$(\Delta x_2, \Delta y_2, \Delta z_2)$

$(\Delta x_m, \Delta y_m, \Delta z_m)$

ORIGINAL

MOTION OF EACH BLOCK WAS CORRECTED

AFTER CORRECTING MOTION

# FIG. 9

$(\Delta x_1, \Delta y_1, \Delta z_1)$

$(\Delta x_m, \Delta y_m, \Delta z_m)$

## FIG. 10A

n FRAME

k FRAME

## FIG. 10B

n FRAME

k FRAME

MECHANICAL ROTATION ANGLE θ

## FIG. 11A

## FIG. 11B

# FIG. 12

START

PERFORM THREE-DIMENSIONAL
SCANNING OPERATION USING MTEE ～S1

CREATE TWO-DIMENSIONAL IMAGE ～S2

EXTRACT IMAGES AT THE SAME TIME PHASE ～S3

CALCULATE MOTION VECTOR
(USING FRAMES AT THE SAME CARDIAC TIME
PHASE AND CLOSE CARDIAC TIME PHASE) ～S4'

PERFORM MOTION CORRECTING PROCESS ～S5

CREATE THREE-DIMENSIONAL IMAGE
(THREE-DIMENSIONAL COORDINATE
TRANSFORMATION/VOLUME RENDERING) ～S6

DISPLAY IMAGE ～S7

END

# FIG. 13

Z
DIRECTION

ROTATION AXIS

# FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5159941 A
- JP 2005074225 A
- US 6966878 B